# EUROPEAN PATENT APPLICATION

(11) **EP 4 353 169 A1**
(43) Date of publication of application: **17.04.2024**
(21) Application number: 23203025.4
(22) Date of filing: 11.10.2023
(51) Int. Cl.: A61B 17/80, A61F 2/28, A61F 2/30, A61F 2/44, B33Y 80/00

(54) **3D PRINTED SEMI-FINISHED PART FOR MEDICAL DEVICE MANUFACTURING AND MEDICAL DEVICES THEREOF**

(30) Priority: 12.10.2022 EP 22201068
(71) Applicant: Kumovis GmbH, 81369 München (DE)
(72) Inventor: Bruyas, Arnaud, 81549 München (DE); Krieger, Yannick, 81541 München (DE); Leonhardt, Stefan, 81677 München (DE); Pammer, Sebastian, 81677 München (DE)
(74) Representative: DTS Patent- und Rechtsanwälte Schnekenbühl und Partner mbB

(57) **Abstract**

The present invention relates to a 3D-printed semi-finished part (100) for manufacturing at least one medical implant or medical instrument, the semi-finished part (100) including:
at least one functional feature (10) provided at a predefined location of the semi-finished part (100).

## Description

The present invention belongs to the field of 3D-printed devices for medical applications, such as 3D-printed medical implants or medical instruments.

Medical implants obtained through 3D printing processing are known in the art and have gained increasing importance in the last decades.

For example, WO2021094624A1 discloses an orthopedic implant obtained through a 3D printing process, said orthopedic implant comprising at least one first portion and at least one second portion, the first portion forming a support structure and providing stiffness and mechanical strength to the implant, and the second portion being at least partially made of a biodegradable material to allow improved ingrowth.

A 3D printing device and method for manufacturing a medical implant is known, e.g., from DE102020126764A1.

In particular, fused filament fabrication (FFF) has proven advantageous in manufacturing of 3D-printed medical implants, as it allows to improve osseointegrative effect and primary stability of the implant.

FFF has also proven effective in manufacturing 3D-printed medical instruments.

3D-printed medical devices, such as medical implants or medical instruments, can be manufactured by using semi-finished parts.

In particular, said semi-finished parts are extruded pieces (e.g., made of a high-performance polymer) that are used to define the outline of the implant.

For instance, semi-finished parts may be in the form of a cube, parallelepiped or cylinder. Alternatively, more complex configurations are also possible.

Several medical devices, such as medical implants or medical instruments, even with slight variations (e.g., in shape or the like), can be post-processed starting from a single semi-finished part.

3D-printed medical implants quality (e.g., distortion, tolerances, strength, toughness etc.) and specific properties (e.g., microstructure or surface properties) is increasingly in the focus of many scientific investigations.

In particular, there is the need for an improved medical implant having enhanced properties (e.g., improved osseointegration, improved osseo conductivity, improved mechanical properties or the like).

There is also the need for a surgical instrument having improved mechanical properties and which can be easily manufactured through 3D-printing.

It is an object of the present invention to provide a solution allowing to obtain a 3D-printed medical device having improved mechanical properties and which can be easily and conveniently manufactured through 3D-printing.

Also, it is an object of the present invention to provide a medical implant having enhanced properties, e.g. in terms of improved osseointegration, improved osseo conductivity, improved bone replacement and/or augmentation mechanical properties or the like.

These objects are achieved by the provision of a 3D-printed semi-finished part according to claim 1 and a 3D-printed medical implant according to claim 10.

According to the invention, a 3D-printed semi-finished part for manufacturing at least one medical device includes:
at least one functional feature provided at a predefined location of the semi-finished part.

The present invention provides a 3D-printed semi-finished part for manufacturing at least one medical device.

For instance, said at least one medical device may include at least one medical implant.

Alternatively, said at least one medical device may include at least one medical instrument.

The 3D-printed semi-finished part can be used for manufacturing a single medical device, such as a medical implant or a medical instrument.

Alternatively, the same 3D-printed semi-finished part can be used for manufacturing a plurality of medical devices, e.g. a plurality of medical implants.

This can be achieved, e.g., by stacking.

The semi-finished part includes at least one functional feature provided at a predefined location of the semi-finished part.

In particular, the location of the at least one functional feature is defined based on the desired properties of the medical device, e.g. a medical implant or medical device, to be manufactured.

A plurality of medical devices, even with slight variations (e.g., in shape or the like), can be post-processed from the 3D-printed semi-finished part.

Further, the 3D-printed semi-finished part can be easily and reliably clamped to a computer numerical control (CNC) machine for implementing a 3D printing process for manufacturing the medical device, e.g. a medical implant or medical instrument.

The at least one medical device, e.g. a medical implant or medical instrument, can be manufactured by implementing conventional 3D printing processes that are known in the art.

The invention is based on the basic idea that, by the provision of at least one functional feature at a predefined location of a 3D-printed semi-finished part, it is possible to obtain an improved medical device having enhanced mechanical properties, and which can be conveniently manufactured though 3D-printing with greater ease. Also, the provision of a 3D-printed semi-finished part as defined above allows obtaining an improved 3D-printed medical implant having enhanced properties (e.g., improved osseointegration, improved osseo conductivity, improved mechanical properties or the like) that cannot be achieved by implementing conventional 3D printing processes alone.

Advantageously, the at least one functional feature may include a porous structure.

By providing at least one porous structure at a predefined location of the semi-finished part, a plurality of pores can be easily formed on one or more desired locations of the medical device, in particular a medical implant, with high precision.

Advantageously, the at least one functional feature can be made of a material that is different than the material of the semi-finished part.

This allows to provide enhanced strength to selected parts of the semi-finished part and/or shape its cosmetic appearance.

In particular, the semi-finished part can be manufactured through fused filament fabrication (FFF).

This allows to obtain a semi-finished part having improved quality.

The at least one functional feature may include at least one clamping feature and/or mounting point.

In particular, said at least one clamping feature and/or mounting point may be configured and adapted to connect the 3D-printed semi-finished part to a computer numerical control CNC machine, e.g. a CNC mill or lathe.

Conveniently, the 3D-printed semi-finished part may have a shape that is configured to at least partially adapt to the shape of the at least one medical device to be manufactured.

For example, the 3D-printed semi-finished part may be shaped so as to match the shape of a patient-specific medical implant to be manufactured.

This allows obtaining a medical device, in particular a medical implant, which better adapts to the patient's anatomy.

The at least one functional feature may also include at least one fixation feature for connecting a medical implant, manufactured by using the 3D-printed semi-finished part, to a target portion within the body of a patient.

For example, said at least one fixation feature may include at least one screw hole.

Also, the present invention provides a 3D-printed medical implant obtained by using a 3D-printed semi-finished part as defined above.

Advantageously, the medical implant includes at least one porous structure.

The porous structure may be configured and adapted to improve osseointegration of the medical implant after implantation at a target site within the body of a patient.

For this purpose, pores of the at least one porous structure may have a pore size between 0,05 mm and 5,0 mm.

Preferably, pores of the at least one porous structure have a pore size between 0,05 mm and 1,0 mm.

The pores may have a circular shape.

Alternatively, the pores may have a rectangular shape.

Alternatively, the pores may have a gyroid shape.

Alternatively, the pores may have a diamond shape.

Alternatively, the pores may have a Schwarz triangle shape.

The pores size and shape are selected based on the desired properties of the medical implant to be manufactured.

The pores of the at least one porous structure may be interconnected.

This allows to further improve osseointegration of the medical implant after implantation at the target site.

Also, the at least one porous structure may be configured and adapted to influence and/or improve mechanical properties of the medical implant.

For this purpose, the at least one porous structure may include at least one portion acting as a spring.

This allows to obtain a medical implant having compliant mechanics, e.g. allowing micro movements to a certain extent.

Also, the at least one porous structure may include at least one portion having non-linear mechanical properties.

This also allows to obtain a medical implant having compliant mechanics.

Also, the at least one porous structure may include at least one portion having elastic properties.

This allows for elastic deformation of the implant after implantation, which enables a certain degree of freedom of movement for the patient.

The at least one porous structure can be provided on one or more surfaces of the implant. This is particularly advantageous in case the at least one porous structure is provided to improve osseointegration.

Advantageously, the 3D-printed medical implant may include a plurality of different materials. Accordingly, it is possible to better tune the properties of the medical implant based on its medical application.

In particular, the medical implant may include at least one high-performance polymer.

In particular, the at least one high-performance polymer may be selected from:
Polyether ether ketone (PEEK);
Polyetherketoneketone (PEKK);
Polyphenylsulfone (PPSU);
Polyaryletherketone (PAEK);
Polyetherketone (PEK);
Polyamide-imide (PAI), or
Polyethylenimine (PEI);

Also, the medical implant may include at least one high-performance polymer comprising one or more ceramic fillers.

For instance, biphasic calcium phosphate (BCP) or bioactive glasses can be used as the ceramic filler.

By the provision of one or more ceramic fillers, it is possible to improve osseo conductivity and osseointegration.

Also, the medical implant may include at least one high-performance polymer comprising one or more fiber reinforcement materials.

By the provision of one or more fiber reinforcement materials at predefined locations of the medical implant, it is possible to better tune the mechanical properties of the medical implant according to its medical application.

Also, the medical implant may include at least one high-performance polymer filled with a radio opaque filler.

By the provision of a radio opaque filler, predefined parts of the medical implant can be made visible in X-Ray/CT.

Also, the medical implant may include at least one drug-loaded material.

By the provision of a drug-loaded material, it is possible to provide area-specific drug delivery to a predefined target area.

Advantageously, the drug may include an anti-inflammatory agent.

Also, the drug may include an antibiotic agent.

The 3D-printed medical implant can be a spinal fusion device, e.g. a spinal cage.

Also, the 3D-printed medical implant can be an osteotomy wedge.

Also, the 3D-printed medical implant can be a total endoprosthesis, e.g. for a knee, hip, shoulder or the like.

Also, the 3D-printed medical implant can be a bone fixation plate.

Also, the 3D-printed medical implant can be a Cranio Maxillo Facial (CMF) implant, e.g. a cranial implant or onlay.

Also, the 3D-printed medical implant can be an augmentation or bone replacement implant. The present invention further provides a 3D-printed medical instrument obtained by using a 3D-printed semi-finished part as defined above.

Further details and advantages of the present invention shall now be disclosed in connection with the drawings, where:

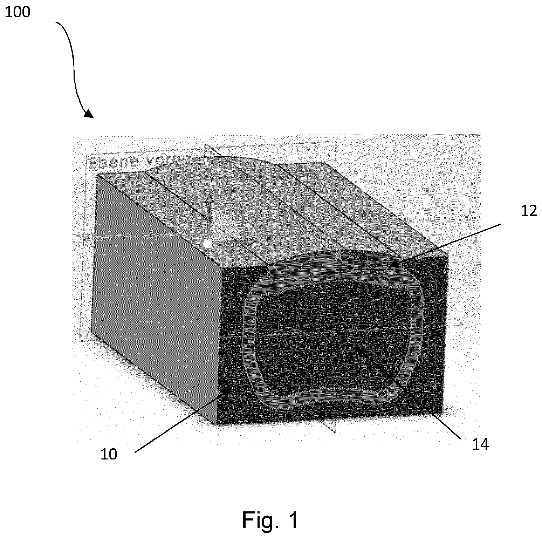

**Fig. 1** is a perspective view of a 3D-printed semi-finished part according to an embodiment of the invention, in particular for manufacturing a spinal cage.

**Fig. 1** shows a 3D-printed semi-finished part 100 for manufacturing at least one medical device, according to an embodiment of the invention.

In the present embodiment, the 3D-printed semi-finished part 100 is configured and adapted for manufacturing a medical implant.

In particular, the semi-finished part 100 is configured and adapted for manufacturing a spinal cage (not shown).

Not shown is that the 3D-printed semi-finished part of the invention may be configured and adapted for manufacturing other kinds of surgical implants.

Not shown is that the 3D-printed semi-finished part of the invention may be configured and adapted for manufacturing a surgical instrument.

The semi-finished part 100 includes a functional feature 10.

Not shown is that the semi-finished part 100 may also include additional functional features, provided at predefined locations of the semi-finished part 100.

In the shown embodiment, the functional feature 10 includes a porous structure 10.

The porous structure 10 is made of a porous material, different than the material of the semi-finished part 100.

In the shown embodiment, the semi-finished part 100 is made of a non-porous material 12.

The porous structure 10 is arranged to surround the non-porous material 12 of the semi-finished part 100.

Further, the porous structure 10 is also arranged to fill a cavity 14 defined by the non-porous material 12 of the semi-finished part 100.

Advantageously, the semi-finished part 100 is manufactured through fused filament fabrication (FFF).

FFF is a 3D printing process which is well-known in the art and which is therefore not further described for the sake of brevity.

Not shown is that the at least one functional feature 10 may include at least one clamping feature and/or mounting point for connecting the 3D-printed semi-finished part 100 to a computer numerical control (CNC) machine, e.g. a CNC mill or lathe.

Conveniently, the 3D-printed semi-finished part may have a shape that is configured to at least partially adapt to the shape of the at least one medical device, in the present case a medical implant, to be manufactured.

In particular, in the present embodiment, the semi-finished part 100 has a shape that is configured to at least partially match the shape of a spinal cage. Not shown is that the at least one functional feature may further include at least one fixation feature for connecting the at least one medical implant, e.g. a spinal fusion device, osteotomy wedge or bone fixation plate, to a target portion within the body of a patient.

For example, said at least one fixation feature may comprise at least one screw hole.

The present invention also provides a 3D-printed medical implant (now shown).

The 3D-printed medical implant can be a spinal fusion device such as a spinal cage or the like.

Alternatively, the 3D-printed medical implant can be an osteotomy wedge.

As a further alternative, the 3D-printed medical implant can be a bone fixation plate.

Also, the 3D-printed medical implant can be a total endoprosthesis, e.g. for a knee, hip or shoulder.

According to yet another alternative, the 3D-printed medical implant can be a Cranio Maxillo Facial (CMF) implant, e.g. a cranial implant or onlay.

According to a still further alternative, the 3D-printed medical implant can be an augmentation or bone replacement implant.

The medical implant is obtained by using a 3D-printed semi-finished part, such as the semi-finished part 100 describe above.

The semi-finished part includes at least one functional feature provided at a predefined location of the semi-finished part.

In one embodiment, the implant includes at least one porous structure.

The at least one porous structure may be adapted for improving osseointegration of the medical implant after implantation.

In such a case, it is preferable that the at least one porous structure is provided on one or more surfaces of the implant.

The pores of the at least one porous structure have a pore size between 0,05 mm and 5,00 mm.

Preferably, the pores of the at least one porous structure have a pore size between 0,05 and 1,0 mm.

The pores of the at least one porous structure have a pore shape selected among circular, rectangular, gyroid, diamond or Schwarz triangle.

In the present embodiment, pores of the at least one porous structure are interconnected.

In an alternative configuration, pores of the at least one porous structure may be non-interconnected.

The porous structure may also be configured and adapted to influence and improve mechanical properties of the medical implant.

For this purpose, the at least one porous structure may include at least one portion acting as a spring, thereby allowing to obtain a medical implant with compliant mechanics.

Additionally or alternatively, the at least one porous structure may include at least one portion having non-linear mechanical properties, also allowing to obtain a medical implant with compliant mechanics.

Additionally or alternatively, the at least one porous structure may include at least one portion having elastic properties that allow for elastic deformation of the implant after implantation, thereby enabling a certain degree of freedom of movement for the patient.

In a further embodiment, optionally in combination with one or more features of the embodiment described above, the medical implant includes a plurality of different materials.

In particular, the medical implant may include at least one high-performance polymer.

Preferably, said high-performance polymer is selected from:
Polyether ether ketone (PEEK);
Polyetherketoneketone (PEKK);
Polyphenylsulfone (PPSU);
Polyaryletherketone (PAEK);
Polyetherketone (PEK);
Polyamide-imide (PAI), or
Polyethylenimine (PEI);

Also, the medical implant may include at least one high-performance polymer comprising one or more ceramic fillers.

For instance, biphasic calcium phosphate (BCP) or bioactive glasses can be used as a ceramic filler.

By the addition of one or more ceramic fillers, it is possible to improve osseo conductivity and osseointegration.

Also, the medical implant may include at least one high-performance polymer comprising one or more fiber reinforcement materials.

By the provision of one or more fiber reinforcement materials at predefined locations of the medical implant, it is possible to better tune the mechanical properties of the medical implant according to its medical application.

Also, the medical implant may include at least one high-performance polymer filled with a radio opaque filler.

By the provision of a radio opaque filler, predefined parts of the medical implant can be made visible in X-Ray/CT.

Also, the medical implant may include at least one drug-loaded material.

By the provision of a drug-loaded material, it is possible to provide area-specific drug delivery to a predefined target area.

Advantageously, the drug may include an anti-inflammatory agent.

Also, the drug may include an antibiotic agent.

Not shown is that the present invention further provides a medical instrument obtained by using a 3D-printed semi-finished part 100 described above.

### References

- 100: 3D-printed semi-finished part
- 10: Functional feature (porous structure)
- 12: Non-porous material
- 14: Cavity

## Claims

1. A 3D-printed semi-finished part (100) for manufacturing at least one medical device, the semi-finished part (100) including:
at least one functional feature (10) provided at a predefined location of the semi-finished part (100).

2. The 3D-printed semi-finished part (100) according to claim 1,
**characterized in that** the at least one functional feature (10) includes a porous structure.

3. The 3D-printed semi-finished part (100) according to claim 1 or 2,
**characterized in that** the at least one functional feature (10) is made of a material that is different than the material of the semi-finished part (100).

4. The 3D-printed semi-finished part (100) according to any one of the preceding claims,
**characterized in that** the semi-finished part (100) is manufactured through fused filament fabrication (FFF).

5. The 3D-printed semi-finished part (100) according to any one of the preceding claims,
**characterized in that** the at least one functional feature (10) includes at least one clamping feature and/or mounting point, configured and adapted to connect the 3D-printed semi-finished part (100) to a computer numerical control (CNC) machine.

6. The 3D-printed semi-finished part (100) according to any one of the preceding claims,
**characterized in that**
said 3D-printed semi-finished part (100) has a shape that is configured to at least partially adapt to the shape of the at least one medical device to be manufactured.

7. The 3D-printed semi-finished part (100) according to any one of the preceding claims,
**characterized in that**
said at least one medical device includes at least one surgical implant.

8. The 3D-printed semi-finished part (100) according to claim 7,
**characterized in that**
the at least one functional feature (10) includes at least one fixation feature for connecting the at least one medical implant, manufactured by using the 3D-printed semi-finished part (100), to a target portion within the body of a patient,
preferably wherein said at least one fixation feature includes at least one screw hole.

9. The 3D-printed semi-finished part (100) according to any one of the preceding claims,
**characterized in that**
said at least one medical device includes at least one surgical instrument.

10. A 3D-printed medical implant obtained by using a 3D-printed semi-finished part (100) according to any one of claims 1 to 8.

11. The 3D-printed medical implant according to claim 10,
**characterized in that** the medical implant includes at least one porous structure.

12. The 3D-printed medical implant according to claim 11,
**characterized in that** pores of the at least one porous structure have a pore size between 0,05 mm and 5,0 mm, preferably between 0,05 and 1,0 mm.

13. The 3D-printed medical implant according to claim 11 or 12,
**characterized in that** pores of the at least one porous structure have a pore shape selected among:
circular;
rectangular;
gyroid;
diamond, or
Schwarz triangle.

14. The 3D-printed medical implant according to any one of claims 10 to 13,
**characterized in that** pores of the at least one porous structure are interconnected.

15. The 3D-printed medical implant according to any one of claims 10 to 14,
**characterized in that** the at least one porous structure includes one or more among:
at least one portion acting as a spring;
at least one portion having non-linear mechanical properties, and/or
at least one portion having elastic properties that allow for elastic deformation of the implant after implantation.

16. The 3D-printed medical implant according to any one of claims 10 to 15,
**characterized in that** the at least one porous structure is provided on one or more surfaces of the implant.

17. The 3D-printed medical implant according to any one of claims 10 to 16,
**characterized in that** the medical implant includes a plurality of different materials.

18. The 3D-printed medical implant according to claim 17,
**characterized in that** said plurality of different materials is selected from:
at least one high-performance polymer, preferably wherein said high-performance polymer is selected from:
Polyether ether ketone (PEEK);
Polyetherketoneketone (PEKK);
Polyphenylsulfone (PPSU);
Polyaryletherketone (PAEK);
Polyetherketone (PEK);
Polyamide-imide (PAI), or
Polyethylenimine (PEI);
at least one high-performance polymer comprising one or more ceramic fillers, preferably wherein the one or more ceramic fillers include biphasic calcium phosphate (BCP) or bioactive glasses;
at least one high-performance polymer comprising one or more fiber reinforcement materials;
at least one high-performance polymer filled with a radio opaque filler, and/or
at least one drug-loaded material, preferably wherein said drug includes an anti-inflammatory agent and/or an antibiotic agent.

19. The 3D-printed medical implant according to any one of claims 10 to 18,
**characterized in that** the medical implant is one among:
a spinal fusion device,
an osteotomy wedge;
a total endoprosthesis;
a bone fixation plate;
a Cranio Maxillo Facial (CMF) implant, and/or
an augmentation or bone replacement implant.

20. A 3D-printed medical instrument obtained by using a 3D-printed semi-finished part (100) according to any one of claims 1 to 6.
